# EUROPEAN PATENT SPECIFICATION

(11) **EP 0 422 922 B1**
(45) Date of publication and mention of the grant of the patent: **28.09.1994**
(21) Application number: 90311115.1
(22) Date of filing: 10.10.1990
(51) Int. Cl.: B65D 83/08, A61F 15/00

(54) **Individual bandage dispenser**
Spender für einzelne Bandagen
Distributeur de pansements individuels

(30) Priority: 10.10.1989 US 418643
(43) Date of publication of application: 17.04.1991
(73) Proprietor: MINNESOTA MINING AND MANUFACTURING COMPANY, St. Paul, Minnesota 55133-3427 (US)
(72) Inventor: Ko, Su-sen, c/o Minnesota Mining & Manuf. Co., St. Paul, Minnesota 55133-3427 (US); Byram, David C., c/o Minnesota Mining & Manuf. Co., St. Paul, Minnesota 55133-3427 (US)
(74) Representative: Baillie, Iain Cameron

(56) References cited:
- US-A- 3 189 219
- US-A- 3 202 316
- US-A- 3 520 403
- US-A- 4 653 666
- US-A- 4 674 634
- US-A- 4 770 320

## Description

### Field of the Invention

This invention relates to an improved dispenser of first aid bandages, and in one aspect, to a stack of first aid bandages joined in a stack to be readily dispensed.

### Background of the Invention

Individual self-adhering bandage strips are well known in the prior art, and are extremely useful for home treatment of minor cuts and abrasions. One such individual bandage product that has enjoyed a great deal of success in this area is the Bandaid Brand Sheer Strip. Individual self-adhering bandages are available in a number of sizes, with a popular size being 19.05m x 76.2m (3/4 inch x 3 inches).

Individual self-adhering bandages are typically sold in individually wrapped units that are provided in boxes of twenty to thirty bandages. These bandages are difficult to dispense and apply due to the packaging arrangements in which they are sold. For example, the individually wrapped bandages are difficult to take out of the box one at a time because they are sufficiently thin that it is hard to grasp one at a time. At times a folded end of one package will catch on another package, further hindering separation of the bandages. Once a bandage has been separated from the rest of the bandages in the box, the wrapper must be removed in order to apply the bandage to the skin. The wrappers are usually designed to be torn apart by pulling on tabs at one end of the package, or by pulling on a little red string that will in turn tear the side of the package. Thus, removing the wrapper from each individual bandage may require a fair amount of finger dexterity. This process is also complicated when the injury to be protected by the bandage is on one of the hands.

Dispensing devices have been suggested into which stacks of bandages can be inserted for individual dispensing of bandages, for example in US-A-3 189 219 and US-A-3 520 403. These devices usually dispense the bandage by urging the bandage from the stack in a direction parallel to the layers of the stack.

U.S. Patent No. 4,674,634 to Wilson discloses a package for dispensing reclosable plastic bags. The bags are arranged in a stack and are adhered one to another along alternately opposite edges of the bags. These bags are placed in a box having an opening in the upper surface of the box with the top bag in the stack extending through the opening. The user withdraws the bag through the opening in the top of the box, and the adhesive will pull the bottom edge of the next successive bag through the opening. The bags may be separated by peeling the adhesive coated edge of the bag dispensed from the next successive bag.

U.S. Patent No. 4,653,666 to Mertens discloses a package for individually dispensing notepapers having a repositionable pressure-sensitive adhesive applied along one of the sheets. These notepapers are adhered one to another along opposite edges of successive sheets, and are dispensed from a card stock box with an opening in the top wall and having polymeric flaps extending into the opening that form an arcuate bend when a notepaper is withdrawn, thus reducing curl of the notepaper.

In US-A-4 770 320, a stack is formed of a stationery product, a major portion of which is covered with a re-positionable adhesive but with an end portion which is non-adhesive with the non-adhesive end portions being alternatively disposed in the stack at opposite ends.

### Summary of the Invention

The present invention relates to an improved method of dispensing individual self-adhering bandages.

A dispenser package is provided for dispensing bandages from a box wherein the bandages are arranged in a stack wherein each of said bandages comprises a backing coated on one side with a pressure sensitive adhesive and an absorbent pad centrally located on said pressure-sensitive adhesive coated side of the backing, said bandages being releasably adhered to each other in the stack, one end portion of the coated side of each bandage releasably adhering to to an end portion of the backing of the successive bandage, on a small part thereof, at alternating opposite ends of successive bandages in the stack. The box has a bottom, side walls and a top wall, with the top wall having a rectangular opening extending generally centrally thereof and defined by opposed parallel margins. A pair of fingers are provided in the top wall so that one finger extends from one margin of the opening and the other finger extends from the opposite margin of the opening. The fingers terminate at terminal edges in an opposed spaced relation and each finger has free side edges movable in relationship to the side walls of the box. The fingers are positioned to normally rest on the top of the stack of bandages, and said fingers have the flexibility to form an arcuate bend transversely of the finger between the margin of the opening and the terminal edge of the finger during dispensing of bandages from the stack and having the resiliency to recover to rest on the top of the stack. Biasing means for urging the stack of bandages toward the opening is provided.

The dispenser is provided with the uppermost bandage in the stack extending through the opening in the top wall. As the user pulls on the free end of the uppermost bandage in the stack and withdraws it from the dispenser, the next successive bandage is pulled through the opening because it is adhesively attached to the distal end of the first bandage. Preferably, the width of the gap between the fingers on the top wall is between about 2 and 6 thicknesses of the thickest portion of the bandage. Most preferably, the gap is between about 2.5 and 4.5 thicknesses of the bandage. The first bandage does not separate from the second because the stress at the adhesive juncture of the bandages is applied in the shear direction. Once the first bandage is totally removed from the dispenser and the second bandage is partially drawn through the opening, the backing of the second bandage contacts a finger, imparting a force in the peel direction on the adhesive between the first and second bandage. The first bandage easily separates from the second bandage, leaving the second bandage in the dispensing position with the free end extending from the dispenser available for grasping by the user.

### Brief Description of the Drawing

The present invention will be further described with reference to the accompanying drawing wherein:
Fig. 1 is an exploded view of the individual bandage dispenser of the present invention.
Fig. 2 is a top view of a bandage dispenser according to the present invention.
Fig. 3 is a side view, partly in section, of a dispenser.
Figs. 4 and 5 show a side view of a stack of bandages stacked in an alternating array according to the present invention.
Figs. 6 and 7 show a section view of a stack of bandages stacked in an alternating array according to the present invention.

### Preferred Embodiments of the Invention

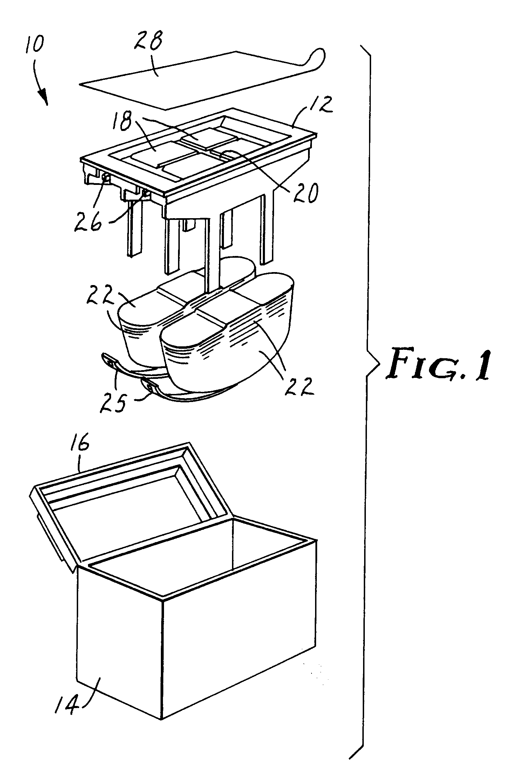

Turning now to the drawing, Fig. **1** shows an exploded view of the individual bandage dispenser of the present invention **10**. Top wall **12** for dispensing individual bandages as shown is slidably received in box **14**. Top wall **12** is preferably anchored in place with respect to box **14** by fastening means. Box **14** is optionally provided with cover **16**, which may be attached to box **14** with a mechanical hinge or a living hinge. Fingers **18** are provided on top wall **12**, with gap **20** having a predetermined width between fingers **18**. Bandages **22** are provided in a stacked formation with each individual bandage adhered to the successive bandage in the stack on alternating ends. Box **14** and fingers **18** are preferably prepared from a plastic material such as styrene or acrylonitrile/butadiene/styrene, or from a polypropylene such as high density polypropylene. Elastomeric tensioning device **25** is attached to top wall **12** at attachment points **26**, thereby urging bandages **22** toward top wall **12**. Tensioning device **25** is preferably prepared from an elastomeric material such as is used to prepare rubber bands. Examples of suitable materials include natural and synthetic rubbers, and especially Kraton rubber. Tensioning device **25** is but one biasing means that may be selected to urge bandages **22** toward top wall **12**. Alternative biasing means include spring-like members made from wire or plastic or a polymeric foam that will exert an upward force on the stack of bandages. Tensioning device **25** is preferred because it provides uniform pressure to the stack of bandages **22** and naturally conforms to the bowed shape of the stack that results from having a centrally located absorbent pad on the bandage. Seal **28** may optionally be provided on top of top wall **12** in order to provide a sterile package unit to the consumer. The user gains access to the bandages by opening cover **16** and peeling off seal **28**, which is then discarded.

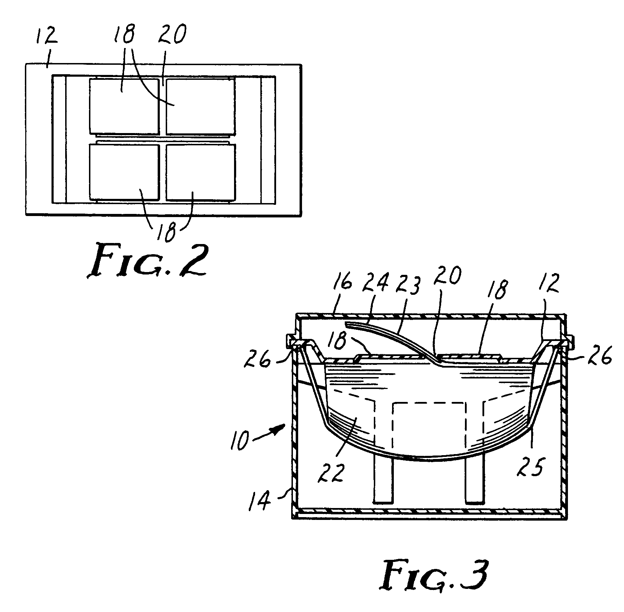

Fig. **2** is a top view of top wall **12**, having two sets of fingers **18** arranged in a parallel array. Fingers **18** are arranged in opposing pairs with predetermined gap **20** between fingers **18**. More than one set of fingers may be provided in order to increase the number of bandages that may be effectively dispensed from a single box. Alternatively, multiple sets of fingers make it possible to dispense bandages of different sizes. Fingers **18** may be integrally formed with top wall **12**, as would result from a one-piece molding process, or may be separately adhered to top wall **12** with a pressure-sensitive adhesive, a hot melt adhesive, or the like. Fingers **18** may vary in size and shape, depending on the flexibility and friction properties desired. For example, fingers **18** may be of any width, but preferably are as wide as the bandage to be dispensed. Fingers **18** additionally may be straight or tapered, either in width or thickness. Fingers **18** may also have a smooth texture imparted to their surfaces, or may have a rough texture to moderate the amount of friction observed between the bandages and the underside of fingers **18**.

Fig. **3** is a side view, partly in section, of dispenser **10**. First bandage **23** having free end **24** extends through gap **20** and is available for grasping by the user. Preferably, attachment points **26** for tensioning device **25** are, as shown, above the level of top wall **12**. In this configuration, tension is provided for even the last bandage in the stack as it progresses up through top wall **12**.

Four factors together should be arranged such that the force required to withdraw a bandage from the dispenser is less than the shear strength of the adhesive interface between bandages, but greater than the peel strength of the adhesive interface between bandages. These four factors are (a) gap between the fingers, (b) finger flexibility, (c) force being applied by the biasing means, and (d) friction between the bandages and the underside of the fingers. All four factors act in harmony, and adjustments in one factor will modify the action of the others.

For illustrative purposes, modification of each of these four factors will be discussed in terms of how it will affect selection of appropriate gap width. It will be understood that such an analysis may now be conducted with respect to each of the identified factors.

When the fingers are very flexible, their ability to hold the next successive bandage in place in the dispenser is limited and the likelihood of multiple bandages being dispensed increases. To compensate for very flexible fingers, the gap between the fingers should be reduced. Similarly, when the amount of force being applied by the tensioning device in urging the bandages upward toward the fingers is low, the likelihood of multiple bandages being dispensed increases. To compensate for a low amount of force being provided by the tensioning device, the gap between the fingers should be reduced. Finally, when there is a low amount of friction between the bandages and the underside of the fingers, bandages will tend to slip out of the dispenser easily with the possibility of multiple dispensing of bandages. The texture of the underside of the fingers should be selected to match the particular material selected for use as the backing of the bandage. For example, a foam-type bandage backing will tend to stick to an extremely smooth surface, particularly when these surfaces contact over a period of time. To reduce the likelihood of sticking in this case, the underside of the fingers may be imparted with a series of ribs running in the travel direction of the bandage. In order to avoid sticking, but increase friction in removal of the bandage, the ribs may be provided perpendicular to the direction of travel of the bandage. When the bandage backing is made from a material that does not exhibit flow characteristics, a smooth texture on the underside of the fingers will result in very low friction interaction. In this case, higher friction may be provided by imparting a rough texture to the underside of the fingers, either as a random pattern or in a series of grooves, ribs or shapes such as a diamond pattern. In the alternative, the backing of the bandage may be surface treated or coated to enhance the slipperiness or stickiness of the backing. With respect to selection of proper gap width, a low-friction interaction between the bandages and the underside of the fingers may be compensated by reducing the distance of the gap between the fingers.

Typically, an appropriate selection of gap width will be from about 2 to 6 thicknesses of bandage. Preferably, the gap width will be from about 2.5 to 4.5 thicknesses of bandage.

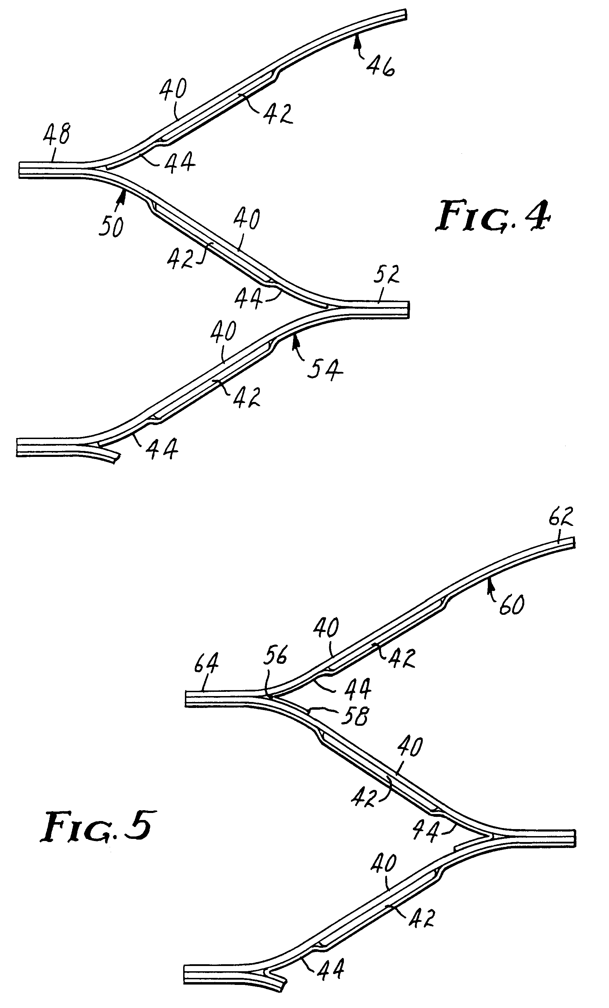

The various possible arrays of bandages are shown in Figs. **4-7**. The typical individual bandage comprises adhesive coated backing **40** with absorbent pad **42** centrally located thereon, and with liner **44** covering the adhesive coated side of backing **40**. As shown in Fig. **4** the liner **44** of first bandage **46** is shortened so that distal end **48** of first bandage **46** extends beyond liner **44** and releasably adheres to the backing of second bandage **50**. Similarly, liner **44** of second bandage **50** is short, so that the distal end **52** of second bandage **50** is releasably adhered to third bandage **54**, and so on. Fig. **5** shows a similar array of bandages, except that instead of liner **44** being short, liner **44** is folded back at fold **56** to form tab **58**. In the removal of first bandage **60**, the user grasps free end **62** and removes bandage **60** from the dispenser **10**. The thus removed bandage **60** has exposed adhesive at end **64** which may be located on the skin of the user. The user then grasps tab **58** and peels off liner **44** from bandage **60**, simultaneously applying bandage **60** to the wound. This embodiment is particularly advantageous because only one hand is required to manipulate the bandage once removed from the box. If the box is additionally anchored in place, complete application of a bandage may be accomplished using only one hand.

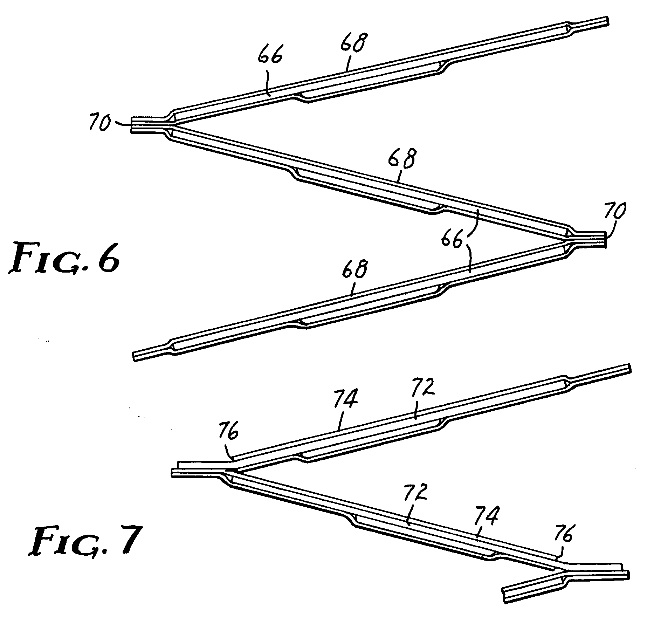

Fig. **6** shows a section view of an alternative bandage configuration with bandages **66** completely enveloped in wrapper **68**. Wrapper **68** of each bandage is adhered to the wrapper of the successive bandage along alternating ends with adhesive **70**. This configuration allows bandages **66** to be provided as individually sterilized units.

Fig. **7** shows a section view of an alternative wrapper configuration, wherein wrapper **74** acts as a release liner for bandage **72**. No separate release liner is provided. Wrapper **74** envelopes bandage **72** and terminates at point **76** allowing the adhesive of bandage **72** to adhere to the wrapper of the next successive bandage.

## Claims

1. A stack of bandages (22) wherein each of said bandages comprises a backing (40) coated on one side with a pressure-sensitive adhesive and an absorbent pad (42) centrally located on said pressure-sensitive adhesive coated side of the backing, said bandages being releasably adhered to each other in the stack, one end portion of the coated side of each bandage releasably adhering to an end portion of the backing of the successive bandage, on a small part thereof, at alternating opposite ends of successive bandages in the stack.

2. A stack of bandages according to claim 1 wherein said bandages are individually wrapped in a bandage wrapper (68) and are releasably adhered to each other in the stack along opposite ends of successive bandage wrappers by a narrow band (70) of pressure-sensitive adhesive.

3. A stack of bandages according to claim 1 wherein said pressure-sensitive adhesive coated surface of said backing and said absorbent pad is partly covered by a liner (44) and having one end (48) of said pressure-sensitive adhesive coated surface of said backing exposed, said bandages being releasably adhered to each other in the stack along opposite ends of said bandages by the pressure-sensitive adhesive of one bandage adhering to an end portion of the backing of the successive backing.

4. A stack of bandages according to claim 3 wherein said liner (44) is folded back onto itself at the end of the bandage having the pressure-sensitive adhesive coated surface of the backing exposed, thereby providing a tab (58) to assist in removing said liner from said bandage after positioning of the exposed pressure-sensitive adhesive surface on the skin.

5. A dispenser package for dispensing bandages,
said package comprising:
the stack (22) of bandages of one of claim 1, 2, 3 or 4;
a box (14) having a bottom, side walls and a top wall (12), said top wall having a rectangular opening extending generally centrally thereof and defined by opposed parallel margins;
a pair of fingers (18), said fingers being disposed for one finger to extend from one margin of said opening and the other finger to extend from the opposite margin of said opening, said fingers terminating at terminal edges in opposed spaced relation (20) and each finger having free side edges movable in relationship to the side walls of the box, said fingers being positioned to normally rest on the top of said stack (22) and said fingers having the flexibility to form an arcuate bend transversely of the finger between the margin of the opening and the terminal edge of the finger during dispensing of bandages from said stack and having the resiliency to recover to rest on the top of the stack, and
biasing means (25) for urging the stack of bandages toward the opening.

6. A dispenser of claim 5 wherein said biasing means comprises a tensioning device (25) attached at the top wall on opposite ends of said bandages.

7. A dispenser of claim 6 wherein said tensioning device (25) is made from an elastomeric material.

8. A dispenser of claim 5 wherein the distance between said opposed fingers (18) is between 2.5 and 4.5 times the thickness of the thickest portion of said bandage.

9. A dispenser of claim 5 having two sets of fingers (18) in a parallel array to deliver two stacks of bandages.

## Patentansprüche

1. Stapel von Bandagen (22), bei dem jede der Bandagen einen auf einer Seite mit einem Haftkleber beschichteten Träger (40) und ein saugfähiges Kissen (42) umfaßt, das mittig auf der mit dem Haftkleber beschichteten Seite des Trägers angeordnet ist, wobei die Bandagen in dem Stapel lösbar aneinander befestigt sind, und wobei ein Endabschnitt der beschichteten Seite jeder Bandage an einem kleinen Stück abwechselnd an den entgegengesetzten Enden der jeweils nächsten Bandagen in dem Stapel lösbar an einem Endabschnitt des Trägers der nächstfolgenden Bandage haftet.

2. Stapel von Bandagen nach Anspruch 1, bei dem die Bandagen einzeln in eine Bandagenhülle (68) eingewickelt sind und in dem Stapel an entgegengesetzten Enden aufeinanderfolgender Bandagenhüllen durch einen schmalen Haftklebestreifen (70) lösbar aneinander befestigt sind.

3. Stapel von Bandagen nach Anspruch 1, bei dem die mit Haftkleber beschichtete Oberfläche des Trägers und das saugfähige Kissen zum Teil mit einer Folie (44) bedeckt sind, und bei dem ein Ende (48) der mit Haftkleber beschichteten Oberfläche des Trägers freiliegt, wobei die Bandagen in dem Stapel an ihren entgegengesetzten Enden dadurch lösbar aneinander befestigt sind, daß der Haftkleber einer Bandage an einem Endabschnitt des Trägers der nächstfolgenden Bandage haftet.

4. Stapel von Bandagen nach Anspruch 3, bei dem die Folie (44) an dem Ende der Bandage, wo die mit Haftkleber beschichtete Oberfläche des Trägers freiliegt, zu sich selbst umgeklappt ist, so daß eine Lasche (58) entsteht, mit der nach dem Positionieren der freiliegenden, mit Haftkleber beschichteten Oberfläche auf der Haut die Folie leichter von der Bandage abgezogen werden kann.

5. Spenderpackung zur Abgabe von Bandagen, wobei die Packung folgendes umfaßt:
den Stapel (22) von Bandagen nach einem der Ansprüche 1, 2, 3 oder 4;
einen Behälter (14) mit einem Boden, Seitenwänden und einer oberen Wand (12), wobei die obere Wand eine rechteckige Öffnung besitzt, die sich im allgemeinen in der Mitte befindet und durch einander gegenüberliegende parallele Ränder begrenzt ist;
ein Paar Finger (18), wobei die Finger so angeordnet sind, daß ein Finger von dem einen Rand der Öffnung und der andere Finger von dem gegenüberliegenden Rand der Öffnung ausgeht, wobei die Finger im Abstand einander gegenüberliegend (20) an Außenkanten enden und jeder Finger freie Seitenkanten besitzt, die in bezug auf die Seitenwände des Behälters beweglich sind, wobei die Finger so positioniert sind, daß sie normalerweise auf der Oberseite des Stapels (22) aufliegen, und so flexibel sind, daß sie sich zwischen dem Rand der Öffnung und der Außenkante des Fingers während der Abgabe von Bandagen aus dem Stapel quer zu dem Finger biegen, und so elastisch sind, daß sie anschließend wieder auf der Oberseite des Stapels aufliegen, und
eine Vorspanneinrichtung (25), die den Stapel von Bandagen in Richtung zu der Öffnung drückt.

6. Spender nach Anspruch 5, bei dem die Vorspanneinrichtung eine Spannvorrichtung (25) umfaßt, die an der oberen Wand an entgegengesetzten Enden der Bandagen befestigt ist.

7. Spender nach Anspruch 6, bei dem die Spannvorrichtung (25) aus einem elastomeren Material besteht.

8. Spender nach Anspruch 5, bei dem der Abstand zwischen den gegenüberliegenden Fingern (18) das 2,5- bis 4,5-fache der Dicke des dicksten Abschnitts der Bandage beträgt.

9. Spender nach Anspruch 5 mit zwei Sätzen von Fingern (18) in einer parallelen Anordnung, so daß zwei Stapel von Bandagen abgegeben werden können.

## Revendications

1. Empilement de pansements (22), dans lequel chacun des pansements comprend un support (40) recouvert sur un côté d'un adhésif sensible à la pression et un tampon absorbant (42) placé de façon centrale sur ledit côté du support recouvert d'adhésif sensible à la pression, lesdits pansements adhérant l'un à l'autre de façon détachable dans l'empilement, une partie d'extrémité du côté recouvert de chaque pansement adhérant de façon détachable à une partie d'extrémité du support du pansement suivant, sur une petite partie de celui-ci, à des extrémités opposées qui alternent de pansements successifs dans l'empilement.

2. Empilement de pansements selon la revendication 1, dans lequel lesdits pansements sont emballés individuellement dans un emballage (68) de pansement et adhèrent l'un à l'autre de façon détachable dans l'empilement le long d'extrémités opposées d'emballages de pansements consécutifs au moyen d'une bande étroite (70) d'adhésif sensible à la pression.

3. Empilement de pansements selon la revendication 1, dans lequel ladite surface recouverte d'adhésif sensible à la pression dudit support et ledit tampon absorbant sont partiellement recouverts d'un revêtement (44) et une extrémité (48) de ladite surface dudit support recouverte d'adhésif sensible à la pression étant à nu, lesdits pansements adhérant l'un à l'autre de façon détachable dans l'empilement le long d'extrémités opposées desdits pansements au moyen de l'adhérence de l'adhésif sensible à la pression d'un pansement à une partie d'extrémité du support du pansement suivant.

4. Empilement de pansements selon la revendication 3, dans lequel ledit revêtement (44) est replié sur lui-même à l'extrémité du pansement dont la surface du support recouverte d'adhésif sensible à la pression est à nu, formant ainsi une patte (58) pour aider à retirer ledit revêtement dudit pansement après application sur la peau de la surface recouverte d'adhésif sensible à la pression.

5. Emballage distributeur pour distribuer des pansements, ledit emballage comprenant :
l'empilement (22) de pansements selon l'une des revendications 1, 2, 3 ou 4 ;
une boîte (14) ayant un fond, des parois latérales et une paroi supérieure (12), ladite paroi supérieure présentant une ouverture rectangulaire située globalement en son centre et délimitée par des bords parallèles opposés ;
une paire de pattes (18), lesdites pattes étant disposées de telle façon qu'une patte s'étend à partir du bord de ladite ouverture et que l'autre patte s'étend à partir du bord opposé de ladite ouverture, lesdites pattes se terminant à des bords terminaux dans une position espacée face à face (20) et chaque patte présentant des bords latéraux libres mobiles par rapport aux parois latérales de la boite, lesdites pattes étant disposées de façon à s'appuyer normalement sur le sommet dudit empilement (22) et lesdites pattes étant suffisamment flexibles pour former une courbure en arc transversalement à la patte entre le bord de l'ouverture et le bord terminal de la patte pendant la distribution de pansements à partir dudit empilement et présentant une élasticité nécessaire pour reprendre leur forme afin de s'appuyer sur le sommet de l'empilement, et
un moyen de poussée (25) pour pousser l'empilement de pansements vers l'ouverture.

6. Distributeur selon la revendication 5, dans lequel ledit moyen de poussée comprend un dispositif tendeur (25) fixé à la paroi supérieure sur les extrémités opposées desdits pansements.

7. Distributeur selon la revendication 6, dans lequel ledit dispositif tendeur (25) est réalisé dans un matériau élastomère.

8. Distributeur selon la revendication 5, dans lequel la distance entre lesdites pattes opposées (18) est comprise entre 2,5 et 4,5 fois l'épaisseur de la partie la plus épaisse dudit pansement.

9. Distributeur selon la revendication 5, comprenant deux ensembles de pattes (18) dans une matrice parallèle pour délivrer deux empilements de pansements.
